# EUROPÄISCHE PATENTSCHRIFT

(11) **EP 0 406 387 B1**
(45) Veröffentlichungstag und Bekanntmachung des Hinweises auf die Patenterteilung: **18.11.1993**
(21) Anmeldenummer: 90901723.8
(22) Anmeldetag: 18.01.1990
(51) Int. Cl.: A61M 39/00

(54) **KUPPLUNG FÜR DIE VERBINDUNG VON SCHLAUCHLEITUNGEN FÜR MEDIZINISCHE ZWECKE**
COUPLING FOR JOINING FLEXIBLE TUBING FOR MEDICAL PURPOSES
RACCORDS DE CONDUITS TUBULAIRES A USAGE MEDICAL

(30) Priorität: 19.01.1989 AT 104/89
(43) Veröffentlichungstag der Anmeldung: 09.01.1991
(73) Patentinhaber: DIERINGER, Franz A., A-1040 Wien (AT)
(72) Erfinder: DIERINGER, Franz A., A-1040 Wien (AT)
(74) Vertreter: Haffner, Thomas M., Dr.
(86) Internationale Anmeldenummer: AT9000008
(87) Internationale Veröffentlichungsnummer: WO9007953

(56) Entgegenhaltungen:
- EP-A- 0 080 379
- EP-A- 0 116 986
- AT-B- 386 523
- US-A- 4 752 292
- US-A- 4 781 702

## Beschreibung

Die Erfindung bezieht sich auf eine Kupplung für die Verbindung von Schlauchleitungen für medizinische Zwecke, mit einem ersten Kupplungsteil, in welchem ein axial verschieblicher hohler Dorn für den Anschluß eines ersten Schlauchstückes gelagert ist, welcher in der Betriebslage einen mit Desinfektionsmittel trankbaren Schwamm durchsetzt und zur Kupplung mit einem zweiten Schlauchstück durch eine Membran durchstechbar ist, welche mit einem zweiten Kupplungsteil verbunden ist, wobei die beiden Kupplungsteile lösbar miteinander verbunden sind.

Eine Einrichtung der eingangs genannten Art ist aus der AT-A-386 523 bekanntgeworden. Bei der bekannten sterilen Verbindung wird ein Dorn axial verschiebbar in einem Kupplungsteil gehalten, und das freie, dem Schlauchanschluß abgewandte Ende des Dornes bewegt sich bei der axialen Verschiebung des Dornes durch elastisch verformbare Einlagen, insbesondere Schaumstoffkörper, hindurch, welche mit Desinfektionsmittel imprägniert werden. Bei der bekannten Ausbildung wird hiebei in vorteilhafter Weise ein mehrteiliger Einsatzteil für die Aufnahme des Desinfektionsmittels aus Schaumstoff vorgesehen, so daß eine Mindestmenge an Desinfektionsmittel ständig in diesem Kupplungsteil vorrätig gehalten werden kann und die erforderliche Menge an Desinfektionsmittel bei mehrmaligem Gebrauch der Einrichtung durch den zweiten herausnehmbaren Schaumstoffkörper ergänzt werden kann. Die Schaumstoffkörper haben hiebei eine zur Achse des Dornes konzentrische Öffnung und werden bei axialer Verschiebung des Dornes unter elastischer Vorspannung gegen die Außenseite des Dornes gepreßt. Bei der bekannten Ausbildung hat der zweite, mit diesem ersten Kupplungsteil verbindbare Kupplungsteil lediglich die Funktion, die an diesen Kupplungsteil angeschlossenen Einrichtungen, wie beispielsweise Infusionsflaschen od.dgl., steril zu verschließen, wofür eine durchstechbare Membran mit diesem zweiten Kupplungsteil verbunden ist.

Die mit dem Dorn verbundene Leitung wurde beispielsweise über einen Katheter unmittelbar mit dem Patienten verbunden und konnte in dauernder Verbindung mit einem derartigen Katheter verbleiben, wenn Infusionsflaschen, Beutel od.dgl. gewechselt werden. Zum Wechsel war es hiebei lediglich erforderlich, den Dorn wiederum in seine zurückgezogene Lage zu bewegen, um sicherzustellen, daß während des Wechsels des Infusionsbesteckes od.dgl. die Infektionsgefahr vermindert wird. Der zweite Kupplungsteil konnte mit dem Infusionsbesteck, insbesondere Infusionsflaschen od.dgl., in leitende Verbindung gebracht werden, und beim neuerlichen Ausfahren des verschieblichen Dornes konnte eine offene Leitungsverbindung bei geringster Kontaminationsgefahr erzielt werden. Ein Verschließen der zum Patienten führenden Leitung erfolgte bei der bekannten Ausbildung nicht. Vielmehr endeten die Durchtrittsöffnungen des Dornes, über welche die Leitungsverbindung mit dem an das zweite Kupplungsstück angeschlossenen Schlauchstück hergestellt wurde, bei zurückgezogener Lage des Dornes in dem mit Desinfektionsmittel tränkbaren Schwamm, wodurch zum einen die Gefahr bestand, daß aus diesem Schwamm Desinfektionsmittel in die zum Patienten führende Leitung angesaugt wird und andererseits bei entsprechender Druckdifferenz Körperflüssigkeiten in den Schwamm ausgepreßt werden konnten.

Aus der EP-A-0 080 379 ist eine weitere Kupplung für die Verbindung von Flüssigkeit enthaltenden Systemen bekanntgeworden, wobei in einem der Kupplungsteile ein Ventilelement integriert ist. Dabei wird ein scheibenförmiges Ventilschließglied durch Beaufschlagung mittels Federkraft an einen Ventilsitz gedrückt, so daß in entkoppeltem Zustand ein Austritt von Flüssigkeit vermieden wird. Durch Einführen und Einpassen des komplementären Kupplungsteiles wird über einen Fortsatz das Ventilschließglied aus seiner schließenden Ruhelage entfernt, so daß eine offene Verbindung über die Kupplungsteile hergestellt wird. Das Vorsehen eines bewegbaren Ventilschließgliedes erfordert jedoch eine genaue Passung der Einzelteile sowie eine Herstellung mit geringen Toleranzen, um eine sterile Abdichtung sicherzustellen.

Die Erfindung zielt nun darauf ab, eine Einrichtung der eingangs genannten Art zu schaffen, bei welcher gleichzeitig mit dem Zurückziehen des Dornes zum Wechsel des Infusionsbesteckes ein dichtender Abschluß der zum Patienten führenden Leitung ermöglicht wird. Zur Lösung dieser Aufgabe besteht die erfindungsgemäße Ausbildung im wesentlichen darin, daß der hohle Dorn aus zwei konzentrisch zueinander angeordneten Teilen besteht, wobei die beiden Teile des axial verschieblichen Dornes relativ zueinander drehverschieblich angeordnet sind und daß in axial eingeschobener Lage des Dornes die Durchtrittsöffnung des Dornes, infolge der Drehverschieblichkeit dessen Teile, verschließbar ausgebildet sind. Dadurch, daß der Dorn zweiteilig ausgebildet ist, wird die Möglichkeit geschaffen, entweder durch relative Verdrehung der beiden Dornteile zueinander oder durch axiale Verschiebung eines Dornteiles relativ zum anderen, eine Absperrung des an den Dorn angeschlossenen Leitungsstückes zu erzielen. Hiebei genügt es, die entsprechenden Verbindungsbohrungen so auszubilden, daß sie in der Betriebslage miteinander fluchten, wodurch eine offene Leitungsverbindung ermöglicht wird und in der zurückgezogenen Lage einander in einer Weise überschleifen, daß ein dichtender Abschluß gewährleistet ist. In besonders einfacher Weise kann dies dadurch erzielt werden, daß der Außenteil des Dornes gemeinsam mit dem Innenteil verschiebbar und verdrehbar am Innenteil des Dornes gelagert ist. Bei einer derartigen Ausbildung kann zwischen innerem und äußerem Dornteil. eine leitende Verbindung in einer vorgegebenen Drehlage erzielt werden, wohingegen in einer der Stellung zum Zurückziehen des Dornes entsprechenden Drehlage die leitende Verbindung unterbrochen sein kann. In einfacher Weise wird dies dadurch erreicht, daß der Innenteil des Dornes in axialer Richtung verlaufend Ausnehmungen oder Abflachungen aufweist, in welche radiale Durchbrechungen zum Anschluß des ersten Schlauchstückes münden und daß der Außenteil radiale Durchbrechungen aufweist, welche in einer Drehlage des Außenteiles relativ zum Innenteil in die axialen Ausnehmungen oder Abflachungen des Innenteiles münden. Derartige, in Längsrichtung des Innenteils des Dornes verlaufende Ausnehmungen oder Abflachungen bilden in axialer Richtung verlaufende Leitungskanäle aus, welche je nach Drehlage des Außenteiles des Dornes mit den entsprechenden Durchbrechungen bzw. radialen Durchtrittsöffnungen des Außenteiles des Dornes in fluchtende Lage gebracht werden können, wohingegen in einer von dieser Betriebslage abweichenden Drehlage die radialen Durchtrittsöffnungen des Außenteiles des Dornes vom Material des Innendornes abgedeckt werden können. Bei einer axialen Verschieblichkeit eines Dornteiles relativ zum anderen muß eine entsprechende Umfangsdichtung gewährleistet sein, und es muß beim Einschieben des Außenteiles des Dornes über den Innenteil des Dornes eine Endlage erzielt werden können, bei welcher die Durchtrittsöffnungen des Außenteiles über die Dichtungselemente am Umfang des Innenteiles des Dornes gesperrt werden.

Um die Sicherheit gegen Kontamination und insbesondere auch die Sicherheit gegen Verschleppen des Desinfektionsmittels in die Blutbahn weiter zu erhöhen, ist die Ausbildung mit Vorteil so getroffen, daß im zweiten Kupplungsteil, welcher die durchstechbare Membran trägt, wenigstens eine saugfähige Einlage, insbesondere eine aus Schaumstoff bestehende Ringscheibe, als Abstreifscheibe vorgesehen ist. Eine derartige Abstreifscheibe nimmt gegebenenfalls an der Außenseite des Dornes anhaftendes Desinfektionsmittel auf, und mit Rücksicht auf den Umstand, daß diese Abstreifscheibe im zweiten Kupplungsteil gelagert ist, wird mit dem Austausch des Infusionsbesteckes sichergestellt, daß auch immer wieder frische Abstreifscheiben gemeinsam mit der zu durchstechenden Membran zum Einsatz gelangen. Die Ausbildung kann hiebei in vorteilhafter Weise so getroffen sein, daß die durchstechbare(n) Membran(en), die Abstreifscheibe sowie ggf. die Dichtscheibe in einem mit dem zweiten Kupplungsglied verriegelbaren gemeinsamen Bauteil angeordnet sind, so daß der gemeinsame Austausch der Abstreifscheibe und der Membran gewährleistet werden kann.

Bei der bekannten Ausbildung der Kupplung war das verbleibende Restvolumen an gegebenenfalls in den Leitungen enthaltener Luft zwar vergleichsweise gering, es konnte jedoch auf Grund der Konstruktion der bekannten Bauteile nicht vollständig ausgeschlossen werden, daß geringe Restmengen an Luft in der Leitung verbleiben, welche dann zusammen mit dem über die Schlauchleitung angeschlossenen Medium über den rohrförmigen Dorn in die Blutbahn gelangen können. Um nun auch das Verschleppen derartiger Restmengen an Luft in die Blutbahn mit Sicherheit zu verhindern, ist mit Vorteil die Ausbildung so getroffen, daß der zweite Kupplungsteil einen exzentrisch zum Verschiebeweg des Dornes angeordneten Anschluß für das zweite Schlauchstück und eine koaxial zum D Verschiebeweg des Dornes angeordnete Entlüftungsöffnung aufweist, welche in der Kupplungsstellung vom Dorn verschließbar ist. Beim Anschluß einer neuen Infusionsflasche an einen derartigen zweiten Kupplungsteil kann zunächst das Restvolumen an Luft über diese Entlüftungsöffnung ausgepreßt werden, wobei die zentrische Entlüftungsöffnung in der Betriebslage des Dornes vom Dorn selbst abgedichtet werden kann. Mit Vorteil ist hiefür die Ausbildung so getroffen, daß das stirnseitige Ende des Außenteiles des Dornes einen axialen Dichtfortsatz trägt, welcher in der Kupplungsstellung in die Entlüftungsöffnung des zweiten Kupplungsteiles dichtend eintaucht. Um eine derartige Entlüftung des Mediums beim Anschluß an den zweiten Kupplungsteil zu erleichtern, und insbesondere um zu verhindern, daß das Medium die am zweiten Kupplungsteil vorgesehene Abstreifscheibe tränkt und damit funktionslos macht, ist mit Vorteil die Ausbildung so getroffen, daß der zweite Kupplungsteil zusätzlich zu seiner dem Anschluß für das zweite Schlauchstück abgewandten durchstechbaren Membran und der Abstreifscheibe eine weitere durchstechbare Membran oder eine Dichtscheibe aufweist, welche den Raum, in welchen der Anschluß für das zweite Schlauchstück mündet, von der Abstreifscheibe trennt. Auf diese Weise bleibt die Abstreifscheibe trocken und saugfähig für das gegebenenfalls an der Außenseite des Dornes anhaftende Desinfektionsmittel.

Um eine sichere Verbindung der Teile des Dornes miteinander zu gewährleisten, ist die Ausbildung mit Vorteil so getroffen, daß der Innenteil des Dornes an seinem den Austrittsöffnungen des Außenteiles abgewandten Ende Klauen aufweist, welche eine Basisscheibe des Außenteiles umgreifen. Eine derartige Verbindung der beiden Teile des Dornes miteinander sichert die Verdrehbarkeit des Außenteiles des Dornes am Innenteil des Dornes, ohne die Stabilität der Verbindung in axialer Richtung zu beeinträchtigen. Um gleichzeitig ein hohes Maß an Dichtheit zu erzielen, kann bei einer derartigen Ausbildung im Bereich dieser kraftschlüssigen Verbindung gegen eine axiale Verschiebung eine entsprechende Dichtung vorgesehen sein, wofür vorteilhaft die Ausbildung so getroffen ist, daß der Innenteil des Dornes im Bereich der 5 Klauen eine konische Dichtfläche für eine hohlkonische Gegenfläche in oder nahe dem Basisteil des Außenteiles des Dornes aufweist. Auf diese Weise läßt sich ein zweiteiliger Dorn mit hoher Präzision, guten Dichteigenschaften und leichter Verdrehbarkeit des Außenteiles des Dornes relativ zum Innenteil des Dornes in einfacher Weise herstellen.

Bei Verwendung konischer Dichtflächen besteht allerdings bei häufiger Verwendung die Gefahr eines Verreibens der konischen Dichtflächen, da sowohl für den Innenteil als auch den Außenteil in erster Linie Kunststoffe eingesetzt werden. Ein Verreiben führt zu einer Vergrößerung des Kraftaufwandes auf das Verschwenken des Außenteiles am Innenteil des Dornes, und es ist daher mit Vorteil die Ausbildung so getroffen, daß der Außenteil des Dornes unter Zwischenschaltung einer Ringdichtung, insbesondere einer Lippendichtung, am Konus des Innenteiles gelagert ist. Auf diese Weise wird neben einer exakten Dichtung auch die Leichtgängigkeit der Schwenkbewegung über lange Betriebszeiträume sichergestellt.

Um die Verschwenkung des Außenteiles des Dornes am Innenteil des Dornes zu ermöglichen, ist die Ausbildung mit Vorteil so getroffen, daß der Außenteil einen radialen Betätigungsfortsatz für die Verschwenkung um die Achse des Innenteiles aufweist, wobei der radiale Betätigungsfortsatz in einer Kulissenführung des ersten Kupplungsteiles in axialer Richtung verschiebbar geführt und in vorbestimmter axialer Verschiebelage in Umfangsrichtung des ersten Kupplungsteiles verschieblich geführt ist, wobei die kulissenartige Führung gleichzeitig sicherstellt, daß die Sperrlage, d.h. diejenige Drehlage des Außenteiles relativ zum Innenteil des Dornes, bei welcher der freie Durchfluß durch die Austrittsöffnungen des Außenteiles des Dornes abgesperrt werden soll, zwangsläufig beim Zurückziehen des Dornes erzielt wird. Es wird somit automatisch beim Zurückziehen des Dornes zum Zwecke des Wechsels des Infusionsbesteckes auch ein sicherer Abschluß der mit dem Patienten verbundenen Leitung erzielt.

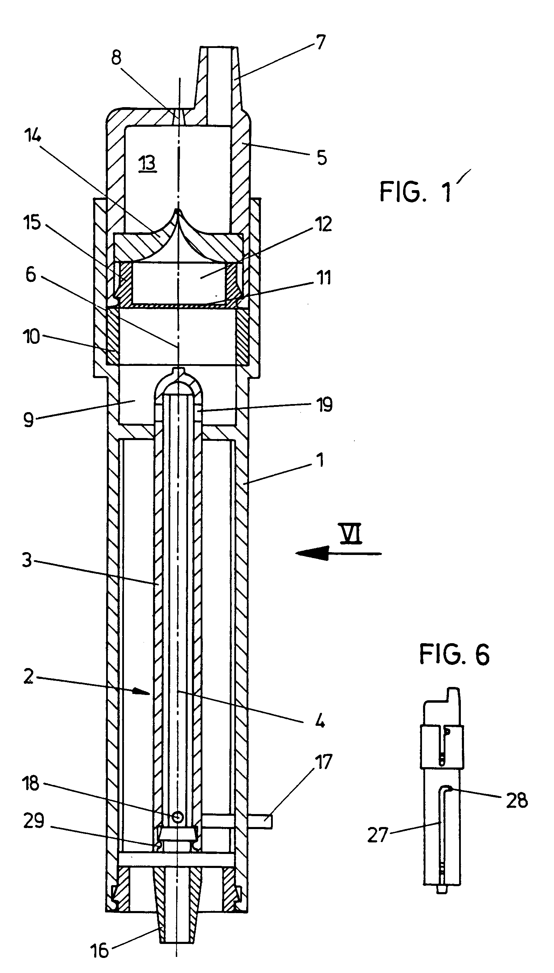

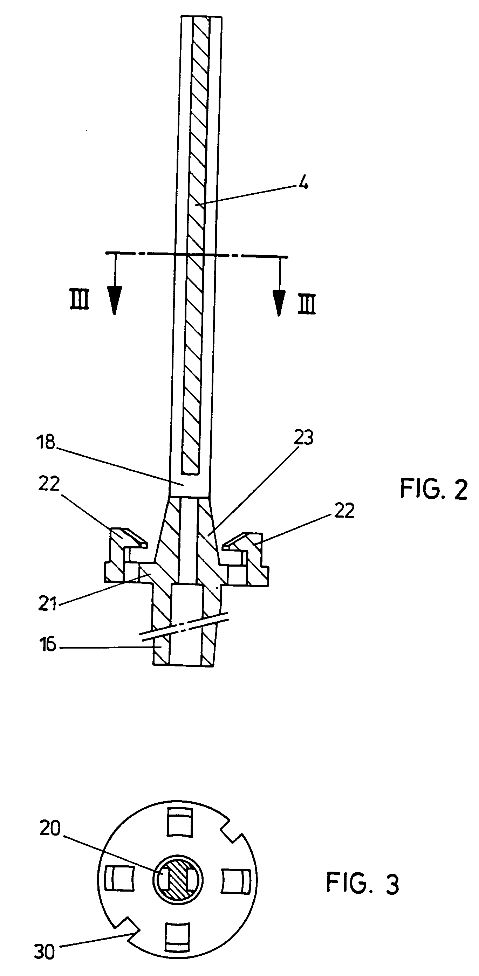

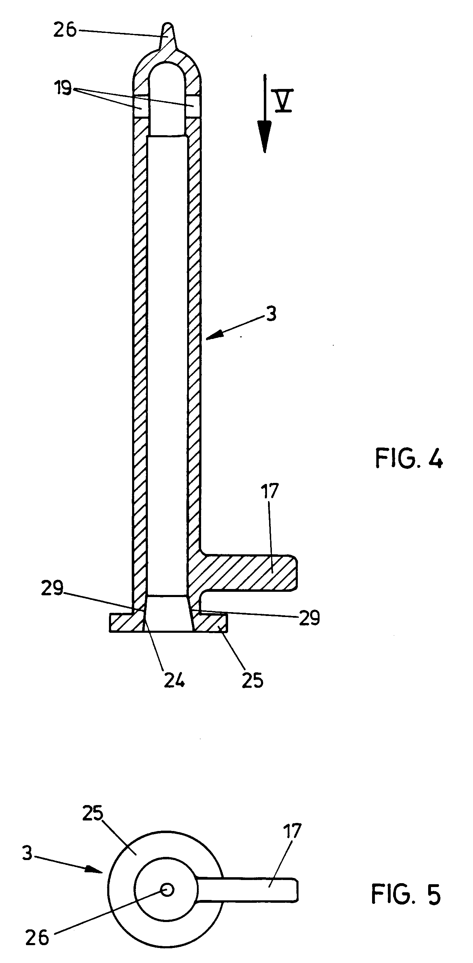

Die Erfindung wird nachfolgend an Hand eines in der Zeichnung schematisch dargestellten Ausführungsbeispieles näher erläutert. In dieser zeigen: Fig. 1 eine schematische Darstellung der erfindungsgemäßen Kupplung im Axialschnitt; Fig. 2 eine vergrößerte Darstellung des Innenteiles des Dornes einer Einrichtung nach Fig. 1; Fig. 3 einen Schnitt nach der Linie III-III der Fig. 2; Fig. 4 einen Axialschnitt durch den Außenteil des Dornes; Fig. 5 eine Draufsicht im Sinne des Pfeiles V der Fig. 4, und Fig. 6 eine verkleinerte Seitenansicht im Sinne des Pfeiles VI der Fig. 1, aus welcher die Kulissenführung für die relative Verdrehung des Außenteiles des Dornes am Innenteil des Dornes ersichtlich ist.

In Fig. 1 ist ein erster Kupplungsteil 1 mit einem in Achsrichtung verschieblichen Dorn 2 dargestellt, wobei dieser Dorn 2 aus einem Außenteil 3 und einem Innenteil 4 besteht. An diesen ersten Kupplungsteil 1 ist ein zweiter Kupplungsteil 5 angeschlossen, an welchen exzentrisch zur Achse 6 des Dornes bzw. des Verschiebeweges des Dornes ein Anschluß 7 für eine weiterführende Schlauchleitung zu einem Infusionsbesteck vorgesehen ist. Zentrisch und innerhalb der Achse 6 ist am zweiten Kupplungsteil 5 eine Entlüftungsbohrung 8 vorgesehen. Bei einer axialen Verschiebung des Dornes 2 wird der Dorn 2 zunächst durch eine erste Einlage 9 aus saugfähigem Material hindurchbewegt, welche permanent mit Desinfektionsmittel getränkt ist. In axialer Richtung anschließend ist eine austauschbare zweite saugfähige Einlage 10 vorgesehen, in welcher das Desinfektionsmittel ergänzt werden kann, um eine sichere Desinfektion zu gewährleisten. Im zweiten Kupplungsteil 5 ist eine durchstechbare Membran 11 sowie eine weitere saugfähige Einlage 12 nach Art einer Abstreifscheibe vorgesehen, welche an der Außenseite des Dornes 2 anhaftendes Material abstreift und aufsaugt.

Beim Anschluß einer Infusionsflasche an den Anschluß 7 kann eine Entlüftung dadurch erzielt werden, daß das eintretende Medium solange in den Raum 13 eingebracht wird, bis es über die Entlüftungsbohrung 8 wiederum abströmt. Um bei einem derartigen Spülvorgang zur Entfernung von Restluft sicherzustellen, daß die Abstreifeinlage 12 nicht mit dem Medium in Berührung gelangt und somit nicht mit Medium getränkt wird, wodurch sie ihre Saugfähigkeit behält, ist eine Dichtungseinlage 14 vorgesehen, welche nach Art einer Lippendichtung einen zentrischen Abschluß bildet. Anstelle einer derartigen Dichtungseinlage 14 kann aber ohne weiteres eine weitere durchstechbare Membran analog der Membran 11 vorgesehen sein, und die Membran 11, die Abstreifscheibe 12 sowie die Dichtungsscheibe 14 sind in einem gemeinsamen Bauteil 15 gehalten, welcher mit dem zweiten Kupplungsteil 5 verrastbar ist.

Um in zurückgezogener Stellung des Dornes, wie dies in Fig. 1 dargestellt ist, sicherzustellen, daß ein dichtender Abschluß der an den Dorn über den Anschluß 16 angeschlossenen, zum Patienten führenden Leitung gewährleistet ist, ist der äußere Teil 3 des Dornes 2 mit einem radialen Betätigungsfortsatz 17 ausgestattet, welcher eine Führung in der Wand des ersten Kupplungsteiles 1 durchsetzt. Der innere Dornteil 4 weist mit dem Anschluß 16 in Verbindung stehende radiale Bohrungen 18 auf, welche in der in Fig. 1 dargestellten Drehlage vom Außenteil 3 des Dornes 2 dichtend umgriffen werden, so daß in der in Fig. 1 dargestellten Lage die radialen Durchbrechungen 19 des Außenteiles 3 des Dornes relativ zu den radialen Durchbrechungen 18 des Innenteiles 4 abgedichtet sind. Dies wird in den Fig. 2 und 3 weiter verdeutlicht.

In Fig. 2 und 3 ist der Innenteil 4 des zweiteiligen Dornes 2 vergrößert dargestellt. Der Innendorn 4 weist, wie aus Fig. 3 hervorgeht, in axialer Richtung verlaufende Kanäle 20 auf, welche in jeweils vorgegebener Drehlage mit den radialen Durchbrechungen 19 des Außenteiles 3 des Dornes in fluchtende Stellung gebracht werden können. Der Innenteil 4 des Dornes ist mit einem Basisteil 21 verbunden, welcher mit Klauen 22 für die Verriegelung des Außenteiles 3 des Dornes 2 ausgestattet ist. Über den Basisteil 21 mündet der Anschluß 16 der zum Patienten führenden Leitung in die radialen Kanäle 18 des Dornes, welche wiederum in den in axialer Richtung verlaufenden Raum münden, welcher von den Ausnehmungen 20 des Innenteiles des Dornes 4 und der Innenwand des Außenteiles 3 des Dornes begrenzt ist. Der Basisteil weist weiters eine konische Dichtfläche 23 auf, welche nach dem Aufstecken des Außenteiles des Dornes in eine durch die Klauen 22 des Basisteiles 21 gegen axiale Verschiebung gesicherte Raststellung eine Dichtfläche zum Außenteil 3 des Dornes ausbilden.

Bei der Darstellung nach den Fig. 4 und 5 ist nun der Außenteil 3 des Dornes vergrößert dargestellt, und es sind wiederum die radialen Durchbrechungen 19 ersichtlich, über welche Medium in die zum Patienten führende Leitung eingebracht werden kann. Hiezu muß allerdings zunächst die Membran 11 des zweiten Kupplungsteiles 5 durchstochen werden und der Dorn über den radialen Betätigungsfortsatz 17 zunächst in axialer Richtung verschoben werden und in der Folge verdreht werden, um die offene Verbindung zwischen den radialen Durchbrechungen 19 und den axial verlaufenden Kanälen 20 des Innendornes herzustellen. Der Außenteil 3 des zweiteiligen Dornes weist an seinem Basisteil wiederum konische Dichtflächen 24 auf, welche bei Aufstecken auf den Innenteil 4 des Dornes mit den konischen Dichtflächen 23 des Innendornes dichtend zusammenwirken. Im gleichen Bereich trägt der Außenteil 3 des Dornes einen Flansch 25, welcher von den Klauen 22 des Innenteiles 4 rastend übergriffen wird, so daß die relative Schwenkbarkeit zwar gewährleistet wird, eine axiale Trennung des Außenteiles 3 vom Innenteil 4 aber nicht ohne weiteres möglich ist.

Das stirnseitige Ende des Außenteiles 3 des Dornes 2 weist einen Dichtungsfortsatz 26 auf, welcher in der Endlage der Verschiebung des Dornes 2 in die Entlüftungsöffnung 8 des zweiten Kupplungsteiles 5 eintaucht und diese dichtend verschließt.

Die Kulissenführung für den radialen Betätigungsfortsatz 17 ist in der Darstellung nach Fig. 6 deutlich ersichtlich. Hiefür ist eine Führungsnut 27 vorgesehen, welche sich über einen ersten Teilbereich in axialer Richtung des ersten Kupplungsteiles 1 erstreckt. Nach Erreichen der Endlage des Führungsdornes, mit welcher die Spitze des Außenteiles 3 über den Dichtungsfortsatz 26 mit der Entlüftungsöffnung 8 zusammenwirkt, kann der radiale Betätigungsfortsatz über einen letzten schrägen Teilbereich 28 der Führungsnut in Umfangsrichtung bewegt werden, wodurch die radialen Durchbrechungen 19 des Außenteiles 3 in eine mit den axialen Kanälen 20 des Innendornes 4 fluchtende Lage verschwenkt werden und die offene Verbindung zwischen den Anschlüssen 7 des Kupplungsteiles 5 und 16 des Dornes 2 hergestellt wird. Gleichzeitig wird der dichtende Abschluß der Entlüftungsöffnung 8 verbessert.

In Fig. 1 und 4 ist ferner eine Ringdichtung 29 ersichtlich, welche mit einer Dichtlippe ausgebildet ist. Eine derartige Ringdichtung 29 stellt die freie Verschwenkbarkeit des Außenteiles 3 des Dornes 2 am Innenteil 4 sicher, ohne daß hiebei der dichtende Abschluß gefährdet wäre.

Ferner ist in Fig. 3 am Umfang des Basisteils eine Ausnehmung 30 ersichtlich. Diese Ausnehmung 30 wirkt mit entsprechend in der Zeichnung nicht dargestellten Fortsätzen am Innenumfang des ersten Kupplungsteiles 1 zusammen und ermöglicht eine Drehsicherung des Innenteiles 4 des Dornes 2, so daß tatsächlich bei einem Verschwenken des Außenteiles 3 durch Betätigen des radialen Betätigungsfortsatzes 17 der Innenteil 4 in seiner Drehlage sicher verbleibt, um die Freigabe der Durchtrittskanäle in der Betriebsstellung zu gewährleisten.

## Patentansprüche

1. Kupplung für die Verbindung von Schlauchleitungen für medizinische Zwecke, mit einem ersten Kupplungsteil (1), in welchem ein axial verschieblicher hohler Dorn (2) für den Anschluß (16) eines ersten Schlauchstückes gelagert ist, welcher in der Betriebslage einen mit Desinfektionsmittel tränkbaren Schwamm (10) durchsetzt und zur Kupplung mit einem zweiten Schlauchstück durch eine Membran (11) durchstechbar ist, welche mit einem zweiten Kupplungsteil (5) verbunden ist, wobei die beiden Kupplungsteile (1,5) lösbar miteinander verbunden sind, dadurch gekennzeichnet, daß der hohle Dorn (2) aus zwei konzentrisch zueinander angeordneten Teilen (3,4) besteht, wobei die beiden Teile (3,4) des axial verschieblichen Dornes (2) relativ zueinander drehverschieblich angeordnet sind und daß in axial eingeschobener Lage des Dornes (2) die Durchtrittsöffnung (19) des Dornes, infolge der Drehverschieblichkeit dessen Teile (3,4), verschließbar ausgebildet ist.

2. Kupplung nach Anspruch 1, dadurch gekennzeichnet, daß der Außenteil (3) des Dornes (2) gemeinsam mit dem Innenteil (4) verschiebbar und verdrehbar am Innenteil (4) des Dornes (2) gelagert ist.

3. Kupplung nach Anspruch 1 oder 2, dadurch gekennzeichnet, daß der Innenteil (4) des Dornes (2) in axialer Richtung verlaufend Ausnehmungen oder Abflachungen (20) aufweist, in welche radiale Durchbrechungen (18) zum Anschluß (16) des ersten Schlauchstückes münden und daß der Außenteil (3) radiale Durchbrechungen (19) aufweist, welche in einer Drehlage des Außenteiles (3) relativ zum Innenteil (4) in die axialen Ausnehmungen oder Abflachungen (20) des Innenteiles münden.

4. Kupplung nach Anspruch 1, 2 oder 3, dadurch gekennzeichnet, daß im zweiten Kupplungsteil (5), welcher die durchstechbare Membran (10) trägt, wenigstens eine saugfähige Einlage, insbesondere eine aus Schaumstoff bestehende Ringscheibe, als Abstreifscheibe (12) aufweist.

5. Kupplung nach einem der Ansprüche 1 bis 4, dadurch gekennzeichnet, daß der zweite Kupplungsteil (5) einen exzentrisch zum Verschiebeweg des Dornes (2) angeordneten Anschluß (7) für das zweite Schlauchstück und eine koaxial zum Verschiebeweg des Dornes (2) angeordnete Entlüftungsöffnung (8) aufweist, welche in der Kupplungsstellung vom Dorn (2) verschließbar ist.

6. Kupplung nach einem der Ansprüche 1 bis 5, dadurch gekennzeichnet, daß der zweite Kupplungsteil (5) zusätzlich zu seiner dem Anschluß (7) für das zweite Schlauchstück abgewandten durchstechbaren Membran (11) und der Abstreifscheibe (12) eine weitere durchstechbare Membran (14) oder eine Dichtscheibe aufweist, welche den Raum (13), in welchen der Anschluß (7) für das zweite Schlauchstück mündet, von der Abstreifscheibe (12) trennt.

7. Kupplung nach einem der Ansprüche 1 bis 6, dadurch gekennzeichnet, daß die durchstechbare(n) Membran(en) (11,14), die Abstreifscheibe (12) sowie ggf. die Dichtscheibe in einem mit dem zweiten Kupplungsglied (7) verriegelbaren gemeinsamen Bauteil angeordnet sind.

8. Kupplung nach einem der Ansprüche 1 bis 7, dadurch gekennzeichnet, daß der Innenteil (4) des Dornes (2) an seinem den Austrittsöffnungen (19) des Außenteiles (3) abgewandten Ende Klauen (22) aufweist, welche eine Basisscheibe (25) des Außenteiles (3) umgreifen.

9. Kupplung nach Anspruch 8, dadurch gekennzeichnet, daß der Innenteil (4) des Dornes (2) im Bereich der Klauen (22) eine konische Dichtfläche (23) für eine hohlkonische Gegenfläche (24) in oder nahe dem Basisteil des Außenteiles (3) des Dornes (2) aufweist.

10. Kupplung nach Anspruch 9, dadurch gekennzeichnet, daß der Außenteil (3) des Dornes (2) unter Zwischenschaltung einer Ringdichtung (29) insbesondere einer Lippendichtung, am Konus (23) des Innenteiles (4) gelagert ist.

11. Kupplung nach einem der Ansprüche 1 bis 10, dadurch gekennzeichnet, daß der Außenteil (3) einen radialen Betätigungsfortsatz (17) für die Verschwenkung um die Achse (6) des Innenteiles (4) aufweist, wobei der radiale Betätigungsfortsatz (17) in eine Kulissenführung (27) des ersten Kupplungsteiles (1) in axialer Richtung verschiebbar geführt und in vorbestimmter axialer Verschiebelage in Umfangsrichtung des ersten Kupplungsteiles (1) verschieblich geführt ist.

12. Kupplung nach einem der Ansprüche 1 bis 11, dadurch gekennzeichnet, daß das stirnseitige Ende des Außenteiles (3) des Dornes (2) einen axialen Dichtfortsatz trägt, welcher in der Kupplungsstellung in die Entlüftungsöffnung (8) des zweiten Kupplungsteiles (5) dichtend eintaucht.

## Claims

1. Coupling for joining flexible tubing for medical purposes, comprising a first coupling part (1) which houses an axially mobile hollow broach (2) for connection to a first piece of flexible tubing (16), which broach penetrates in its operating position a sponge (10) impregnatable with disinfectant and can perforate a membrane (11) for coupling with a second flexible tubing, which is connected with a second coupling part (5), wherein the two coupling parts (1,5) are releasably interconnected, characterized in that the hollow broach (2) consists of two mutually concentric parts (3,4), wherein both parts (3,4) of the axially mobile broach (2) are rotatably slidable relative to each other, and in that in axially inserted position of the broach (2) the passage (19) of the broach (2) is closable, because of the rotatable sliding of the parts (3,4).

2. Coupling according to claim 1, characterized in that the outer part (3) of the broach (2) is supported for being slidable and rotatable on the inner part (4) of the broach (2) together with the inner part (4).

3. Coupling according to claim 1 or 2, characterized in that the inner part (4) of the broach (2) comprises axially extending recesses or flattenings (20) into which open radial openings (18) for connecting (16) the first flexible tube and in that the outer part (3) comprises radial passages (19) opening into the axial recesses or flattenings (20) of the inner part in one rotated position of the outer part (3) relative to the inner part (4).

4. Coupling according to claim 1, 2 or 3, characterized in that at least one adsorbing insert member, in particular an annular disc consisting of foamed material, is provided as a stripper disc (12) within the second coupling part (5) carrying the perforable membrane (10).

5. Coupling according to any of the claims 1 to 4, characterized in that the second coupling part (5) comprises a connecting piece (7) for the second flexible tubing in an excentric position relative to the shifting path of the broach (2) and an air venting opening (8) located coaxially relative to the shifting path of the broach (2), which opening is closable by the broach (2) in the coupled position.

6. Coupling according to any of the claims 1 to 5, characterized in that the second coupling part (5) has, in addition to its perforable membrane (11) located remote from the connecting piece (7) for the second flexible tubing and in addition to the stripper disc (12), a further perforable membrane (14) or a sealing disc which seperates the space (13), into which opens the connecting piece (7) for the second flexible tube, from the stripper disc (12).

7. Coupling according to any of the claims 1 to 6, characterized in that the perforable membrane or membranes (11,14), the stripper disc (12) as well as, optionally, the sealing disc are arranged within a common constructional part which can be locked with the second coupling member (7).

8. Coupling according to any of the claims 1 to 7, characterized in that the inner part (4) of the broach (2) has at its end located remote from the outlet passages (19) of the outer part (3) claws (22) which embrace a base disc (25) of the outer part (3).

9. Coupling according to claim 8, characterized in that the inner part (4) of the broach (2) comprises, within the area of the claws (22), a conical sealing surface (23) for a hollow-conical cooperating surface (24) located within or in proximity of the base member of the outer part (3) of the broach (2).

10. Coupling according to claim 9, characterized in that the outer part (3) of the broach (2) is supported on the cone (23) of the inner part (4) with interposition of an annular sealing (29), in particular a lip seal.

11. Coupling according to any of the claims 1 to 10, characterized in that the outer part (3) has a radial actuating protrusion (17) for being rotated around the axis (6) of the inner part (4), wherein the radial actuating protrusion (17) is guided within a guide slot (27) of the first coupling part (1) for being shiftable in axial direction and is guided for being shiftable in circumferential direction of the first coupling part (1) at a predetermined axially shifted position.

12. Coupling according to any of the claims 1 to 11, characterized in that the front end of the outer part (3) of the broach (2) carries an axial sealing protrusion, which sealingly extends in the coupled position into the air venting opening (8) of the second coupling part (5).

## Revendications

1. Couplage pour la jonction des tuyaux flexibles pour buts médicales, avec un premier élément de couplage (1) dans lequel une axiale déplaçable broche creusée (2) est positionnée pour la connection (16) d'un premier élément de tuyaux flexible, quelle broche traverse dans sa position d'usage une éponge (10) étant imprégnable avec un désinfectant et qui traverse une membrane (11) pour le couplage avec un second élément de tuyaux flexible, qui est joint avec un second élément de couplage (5), à quoi les deux éléments de couplage (1,5) sont résolublement joint, characterisé en ce que la broche creusée (2) se compose de deux éléments (3,4) qui sont rangés concentriquement, à quoi les deux éléments (3,4) de la broche déplaçable axialement sont déplaçable radialement un relative à l'autre et en ce que dans la position axiale insérée de la broche (2) l'ouverture (19) de la broche (2) est fermable, en conséquence du déplacement radial des éléments (3,4).

2. Couplage selon la revendication 1, charactérisé en ce que l' élément extérieur (3) de la broche (2) est positionné avec l'élément intérieur (4) déplacable et pivotable en commun sur l'élément intérieur de la broche (2).

3. Couplage selon l'une des revendications 1 ou 2, charactérisé en ce que l'élément intérieur (4) de la broche (2) a dans la direction axiale des creux ou aplatissements (20), dans lesquels des ouvertures radiales (18) débouchent pour la connection (16) du premier tuyaux flexible et en ce que l'élément extérieur (3) a des ouvertures radiales (19), qui dans une position tournée de l'élément extérieur (3) relative à l'élément intérieur (4) débouchent dans les creux ou aplatissements axiales (20).

4. Couplage selon l'une des revendications 1, 2 ou 3, charactérisé en ce que le second élément de couplage (5), qui porte la membrane (10) traversable, a ou moins un insertion absorbant comme racleur (12), en particulier une disque annulaire constituée de mousse.

5. Couplage selon l'une des revendications 1 à 4, charactérisé en ce que le second élément de couplage (5) a un raccord (7) pour le second élément de tuyaux flexible qui est excentrique à la course de déplacement de la broche (2) et une ouverture d'aérage (8) qui est coaxiale à la course de déplacement de la broche (2), quelle ouverture étant fermable par la broche (2) dans la position de couplage.

6. Couplage selon l'une des revendications 1 à 5, charactérisé en ce que le second élément de couplage (5) a en addition à la membrane (11), qui est opposée au raccord (7) pour le second élément de tuyaux flexible, et le racleur (12) une autre membrane traversable (14) ou une disque étanche, qui separe l'espace (13), dans lequel le raccord (7) pour le second élément de tuyaux flexible se débouche, du racleur (12).

7. Couplage selon l'une des revendications 1 à 6, charactérisé en ce que la (les) membrane(s) traversable(s) (11,14), le racleur (12) et, le cas échéant, la disque étanche sont arrangés dans un élément de construction commun, qui est verrouiable avec le second élément (5) de couplage.

8. Couplage selon l'une des revendications 1 à 7, charactérisé en ce que l'élément intérieur (4) de la broche (2) a des griffes (22) à ses extrémités qui sont départ des ouvertures (19) du élément extérieur (3), quelles griffes enveloppant une disque de base (25) du élément extérieur (3).

9. Couplage selon la revendication 8, charactérisé en ce que l'élément intérieur (4) de la broche (2) a dans la zone des griffes (22) une surface d'étancheité conique (23) pour une contre surface à cone creux (24) en ou prés de l'élément de base de l'élément extérieur (3) de la broche (2).

10. Couplage selon la revendication 9, charactérisé en ce que l'élément extérieur (3) de la broche (2) est logé sur le cone (23) du élément intérieur, par insertion d'une disque étanche (29), en particulier d'un joint à lèvres.

11. Couplage selon l'une des revendications 1 à 10, charactérisé en ce que l'élément extérieur (3) a un prolongement d'actionnement radial (17) pour la rotation autour de l'axe (6) de l'élément intérieur (4), à quoi le prolongement d'actionnement radial (17) est guidé déplaçablement dans la direction axiale dans un guide de coulisse (27) du premier élément de couplage (1) et est guidé déplaçablement dans une position de déplacement prédéterminée axiale en direction péripherique du premier élément de couplage (1).

12. Couplage selon l'une des revendications 1 à 11, charactérisé en ce que l'extrémité frontale de l'élément extérieur (3) de la broche (2) porte un prolongement axial étanche, qui plonge étanchement dans l'ouverture d' aérage (8) du second élément de couplage (5) en position du couplage .
